# EUROPÄISCHE PATENTSCHRIFT

(11) **EP 1 072 577 B1**
(45) Veröffentlichungstag und Bekanntmachung des Hinweises auf die Patenterteilung: **09.03.2005**
(21) Anmeldenummer: 00115006.9
(22) Anmeldetag: 21.07.2000
(51) Int. Cl.: C07C 29/132, C07C 29/141

(54) **Herstellung von Pentandiolen aus Alkoxydihydropyranen**
Preparation of pentanediols from alkoxydihydropyrans
Préparation de pentadioles d'alkoxydihydropyrannes

(30) Priorität: 29.07.1999 DE 19935828
(43) Veröffentlichungstag der Anmeldung: 31.01.2001
(73) Patentinhaber: BASF AKTIENGESELLSCHAFT, 67056 Ludwigshafen (DE)
(72) Erfinder: Pfeffinger, Joachim, 67063 Ludwigshafen (DE); Wahl, Peter, Dr., 68526 Ladenburg (DE); Nouwen, Jan, Dr., 64653 Lorsch (DE); Eller, Karsten, Dr., 67061 Luwigshafen (DE); Höhn, Arthur, Dr., 67281 Kirchheim (DE); Hunger, Jürgen, Dr., 67067 Ludwigshafen (DE)
(74) Vertreter: Isenbruck, Günter, Dr.

(56) Entgegenhaltungen:
- EP-A- 0 394 842
- DE-B- 1 003 704
- US-A- 653 765
- US-A- 2 546 019

## Beschreibung

Die Erfindung betrifft ein Verfahren zur Herstellung von 1,5-Pentandiolen durch einstufige Umsetzung von Alkoxydihydropyranen mit Wasser und Wasserstoff in Gegenwart eines Katalysators sowie die Verwendung des Katalysators in diesem Verfahren.

1,5-Pentandiol ist ein wichtiges Zwischenprodukt zur Herstellung von Polyestern, Polyurethanen sowie von heterocyclischen Verbindungen wie 1-Methylpiperidin, welche als Vorprodukte für die Herstellung von Arznei- und Pflanzenschutzmitteln eingesetzt werden.

Pentandiol wird technisch in großem Maßstab, wie in Ullmann's Encyclopedia of Industrial Chemistry, 5^{th} Edition, Vol. A1, pp. 307 beschrieben, durch katalytische Hydrierung von Glutarsäure, welche industriell als Kuppelprodukt bei der Adipinsäureherstellung anfällt, oder seinen Estern hergestellt. Bei der katalytischen Hydrierung der Adipinsäure/Glutarsäuregemische fällt Pentandiol nur zu circa 10 Mol.-% an. Außerdem ist die Gewinnung von sehr reinem Pentandiol aus diesem Stoffgemisch wegen der sehr ähnlichen Siedepunkte von Pentandiol und Hexandiol aufwendig.

Ein weiteres Verfahren zur Herstellung von Pentandiol geht von Glutardialdehyd aus, welches durch saure Hydrolyse von Alkoxydihydropyranen gewonnen wird. Verfahren zur Hydrierung von Dialdehyden sind in DE-A 4 414 274 beschrieben, wobei auch die Halb- bzw. Vollacetale der Dialdehyde eingesetzt werden können. Als Katalysator wird ein Monolithkatalysator, der ein Edelmetall auf einem mit Aluminium/Siliciumoxid beschichteten Metallträger enthält, eingesetzt. Nachteilig an diesen Verfahren ist, daß ausgehend von Alkoxydihydropyranen eine zweistufige Umsetzung, bestehend aus saurer Hydrolyse und nachfolgender Hydrierung, notwendig ist.

Es hat daher Versuche gegeben, Alkoxydihydropyran in einem einstufigen Verfahren direkt zu Pentandiol umzusetzen. In DE-A 1 003 704 und US 2,546,019 sind Verfahren beschrieben, in denen Pentandiol durch einstufige Umsetzung von Alkoxydihydropyran mit Wasser und Wasserstoff in Gegenwart eines Katalysators erhalten wird, wobei als Hydrierkatalysatoren Metalle wie Pt, Pd, Au, Ag, Zn, V, W, Co, Ni, Ru, Rh, Mn, Cr, Mo, Ir, Os, Pb, deren Legierungen sowie deren Oxide und Sulfide eingesetzt werden können sollen. Als bevorzugte Hydrierkatalysatoren werden pyrophore Metallhydrierungskatalysatoren aus Nickel, Cobalt und Eisen genannt. Diese als Raney-Katalysatoren bekannten Hydrierkatalysatoren können als feinteilige Pulver in der Reaktionslösung dispergiert oder auch in Form größerer Partikel als Festbettkontakt eingesetzt werden. Ferner wird die Möglichkeit erwähnt, den Metallkatalysator auf ein Trägermaterial wie Bimsstein oder Kieselgur aufzubringen. Nachteilig an den feindispersen Raney-Katalysatoren sind deren pyrophore Eigenschaften, die eine Handhabung der Pulver unter Inertgas erfordern, sowie deren Giftigkeit. Die Umsetzungen mit feindispersen Raney-Katalysatoren werden überwiegend diskontinuierlich durchgeführt, wobei die Suspension nach der Umsetzung filtriert werden muß. Die kontinuierliche Durchführung dieser Umsetzungen ist relativ aufwendig, da kontinuierlich filtriert und der am Filter zurückgehaltene Katalysator in den Reaktor zurückgeführt werden muß. Andererseits ist die Herstellung von Raney-Katalysatoren, die sich als Festbettkontakte eignen, technisch aufwendig.

Aufgabe der vorliegenden Erfindung ist es, ein einstufiges Verfahren zur Herstellung von Pentandiolen aus Alkoxydihydropyranen bereitzustellen, welches ohne den Einsatz von Raney-Katalysatoren auskommt.

Gelöst wurde die Aufgabe durch ein Verfahren zur Herstellung von 1,5 Pentandiolen der allgemeinen Formel (I) 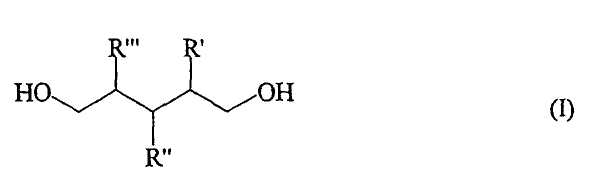 durch einstufige Umsetzung von Alkoxydihydropyranen der allgemeinen Formel (II) 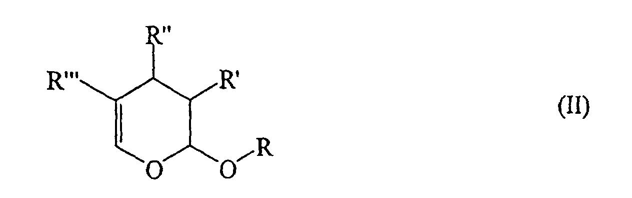 wobei in den allgemeinen Formeln (I) bzw. (II)
R, R', R'', R''' gleich oder verschieden sein können und Wasserstoff oder einen linearen oder verzweigten gesättigten Kohlenwasserstoffrest mit 1 bis 20 C-Atomen, der in der Kohlenwasserstoffkette als Heteroatome O, S und N enthalten kann und mit Hydroxy-, Thiol-, Aminogruppen oder Halogenen einfach oder mehrfach substituiert sein kann,
bedeuten,
mit Wasser und Wasserstoff in Gegenwart eines Katalysators, dadurch gekennzeichnet, daß der Katalysator Oxide von Nickel, Zirkon und Kupfer enthält.

Der erfindungsgemäß eingesetzte Katalysator enthält Oxide von Nickel, Zirkon und Kupfer. Der Katalysator kann außerdem Molybdänoxid enthalten. Vorzugsweise enthält der erfindungsgemäß eingesetzte Katalysator 20 bis 75 Gew.-%, besonders bevorzugt 30 bis 70 Gew.-%, ganz besonders bevorzugt 40 bis 60 Gew.-% und speziell 35 bis 55 Gew.-% Nickeloxid, vorzugsweise 10 bis 75 Gew.-%, besonders bevorzugt 10 bis 60 Gew.-%, ganz besonders bevorzugt 15 bis 50 Gew.-% und speziell 25 bis 45 Gew.-% Zirkondioxid, vorzugsweise 5 bis 50 Gew.-% Kupferoxid, besonders bevorzugt 5 bis 40 Gew.-%, ganz besonders bevorzugt 10 bis 35 Gew.-% und speziell 10 bis 20 Gew.-% Kupferoxid. Der Katalysator kann außerdem bis zu 5 Gew.-% beispielsweise 0,1 bis 5 Gew.-% Molybdänoxid enthalten. Die angegebenen Gewichtsanteile beziehen sich jeweils auf den oxidischen nichtreduzierten Katalysator und ergeben in ihrer Summe 100 Gew.-%.

In einer Ausführungsform enthält der erfindungsgemäß eingesetzte Katalysator Nickeloxid, Zirkondioxid und Kupferoxid und kein Molybdänoxid. In einer weiteren Ausführungsform enthält der erfindungsgemäß eingesetzte Katalysator außerdem 0,1 bis 5 Gew.-% Molybdänoxid. Vorzugsweise enthalten die erfindungsgemäßen Katalysatoren nur die Metalle Nickel, Zirkon, Kupfer und gegebenenfalls Molybdän und daneben weitere Metalle allenfalls in Spuren, beispielsweise in Anteilen von < 1 Mol.-%, bevorzugt < 0,1 Mol.-%, bezogen auf den Gesamtgehalt an Metallen. Bevorzugt sind es somit Katalysatoren, die im wesentlichen aus den oben genannten Metalloxiden in den oben angegebenen Grenzen bestehen.

Im allgemeinen werden die erfindungsgemäß eingesetzten Katalysatoren in Form von Vollkatalysatoren eingesetzt. Mit dem Begriff Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, daß man die katalytisch aktive, zu Pulver vermahlene Masse in das Reaktionsgefäß einbringt oder, daß man die katalytisch aktive Masse nach Mahlung, Vermischung mit Formhilfsmitteln, Formung und Temperung als Katalysatorformkörper - beispielsweise als Kugeln, Zylinder, Ringe und Spiralen - im Reaktor anordnet.

Im allgemeinen werden zur Herstellung der erfindungsgemäß eingesetzten Katalysatoren Fällungsmethoden angewandt. So können sie beispielsweise durch eine gemeinsame Fällung der Nickel- und Kupferkomponenten aus einer dieser Elemente enthaltenden, wäßrigen Salzlösung mittels Mineralbasen in Gegenwart einer Aufschlämmung einer schwerlöslichen, sauerstoffhaltigen Zirkonverbindung und anschließendem Waschen, Trocknen und Caicinieren des erhaltenen Präzipitats erhalten werden. Als schwerlösliche, sauerstoffhaltige Zirkonverbindungen können beispielsweise Zirkondioxid und Zirkonoxidhydrat eingesetzt werden. Molybdän kann vor dem Trocknen als Ammoniumheptamolybdat zugegeben werden.

Die erfindungsgemäß eingesetzten Katalysatoren können über eine gemeinsame Fällung der Nickel- und Kupferkomponenten erhalten werden, wobei die diese Elemente enthaltende, wäßrige Salzlösung in der Wärme und unter Rühren solange mit einer wäßrigen Mineralbase, insbesondere einer Alkalimetallbase wie Natriumcarbonat, Natriumhydroxid, Kaliumcarbonat oder Kaliumhydroxid versetzt wird, bis die Fällung vollständig ist. Die bei diesen Fällungsreaktionen erhaltenen Niederschläge sind im allgemeinen chemisch uneinheitlich und bestehen unter anderem aus Mischungen der Oxide, Oxidhydrate, Hydroxide, Carbonate und unlöslichen basischen Salzen der eingesetzten Metalle. Es kann sich für die Filtrierbarkeit der Niederschläge als günstig erweisen, wenn sie gealtert werden.

Bevorzugt wird der erfindungsgemäß eingesetzte Katalysator dadurch hergestellt, daß man Salze der Metalle Nickel, Kupfer und Zirkon in wäßriger Lösung bei einer Temperatur von 30 bis 90°C und einem pH-Wert von 5 bis 9 ausfällt, die Suspension filtriert und den Filterkuchen trocknet und bei einer Temperatur von 300 bis 700°C tempert, wobei man gegebenenfalls das Molybdän als Ammoniumheptamolybdat vor dem Trocknen zugibt. Die Fällung wird so vorgenommen, daß man eine wäßrige Lösung von Salzen, wie den Nitraten, Sulfaten oder Acetaten der Metalle Nickel, Kupfer und Zirkon mit einer wäßrigen Lösung von Alkalicarbonat mischt. Die Menge der Metallsalze wird dabei so bemessen, daß die Katalysatormasse nach dem Tempern die angegebene Zusammensetzung aufweist.

In einer weiteren bevorzugten Variante wird bei der Herstellung des erfindungsgemäß eingesetzten Katalysators das wasserlösliche Zirkonsalz teilweise, beispielsweise bis zu einem Anteil von 50 Gew.-% bezogen auf das eingesetzte Zirkon, durch festes Zirkondioxid, welches der wäßrigen Metallsalzlösung vor der Fällung zugegeben oder im Reaktionsgefäß vorgelegt wird, ersetzt.

Bei der Herstellung des erfindungsgemäß eingesetzten Katalysators wird beispielsweise die wäßrige Lösung der Metallsalze gleichzeitig unter Rühren mit einer wäßrigen Alkalicarbonatlösung, vorzugsweise Natriumcarbonatlösung, vermischt, wobei die Metalle in Form eines Gemisches von Metallhydroxiden und Metallcarbonaten ausfallen. Der Metallsalzgehalt der Metallsalzlösung beträgt vorzugsweise 30 bis 40 Gew.-%. Die wäßrige Alkalicarbonatlösung ist vorzugsweise 15 bis 20 gew.-%ig.

Die erhaltene Suspension wird filtriert und so lange mit Wasser gewaschen, bis keine Anionen mehr nachgewiesen werden können. Anschließend wird sie bei einer Temperatur von 120 bis 200°C im Trockenschrank oder in einem Sprühtrockner getrocknet. Das Molybdän wird ggf. als Ammoniumheptamolybdat dem feuchten Filterkuchen zugegeben. Der getrocknete Filterkuchen wird bei einer Temperatur von 350 bis 700°C, vorzugsweise 400 bis 600°C getempert. Die so erhaltene Katalysatormasse kann vor dem Einsatz tablettiert oder extrudiert werden. Beispielsweise wird die Katalysatormasse unter Verwendung eines Tablettierhilfsmittels, vorzugsweise Graphit, zu Tabletten mit den Dimensionen 6 x 3 mm verpreßt. Die auf diese Weise hergestellten Tabletten werden bei einer Temperatur von 300 bis 700°C, vorzugsweise 400 bis 600°C, getempert. Die so erhaltenen Tabletten haben im allgemeinen ein Mittelgewicht von 1500 bis 1900 g/l, eine Porosität (durch Wasseraufnahme bestimmt) von 0,2 bis 0,4 ml/g und eine Härte von 3000 bis 4000 N/cm². Der auf diese Weise erhältliche Katalysator wird vor seiner erfindungsgemäßen Verwendung im allgemeinen einer reduktiven Behandlung mit Wasserstoff bei einer Temperatur von 200 bis 350°C, vorzugsweise 230 bis 280°C über einen Zeitraum von beispielsweise 20 bis 40 Stunden bei einem Wasserstoffdruck von im allgemeinen 1 bis 300, vorzugsweise 100 bis 150 bar, unterworfen.

Die erfindungsgemäß eingesetzten Katalysatoren können auch durch Peptisieren pulvriger Mischungen der Hydroxide, Carbonate, Oxide und/oder Salze von Nickel, Zirkon, Kupfer und ggf. Molybdän mit Wasser und nachfolgendem Extrudieren und Tempern der so erhaltenen Massen hergestellt werden.

Die 1,5-Pentandiole der allgemeinen Formel (I) werden durch Umsetzung von Alkoxydihydropyranen der allgemeinen Formel (II) entsprechend der unten stehenden Reaktionsgleichung erhalten.

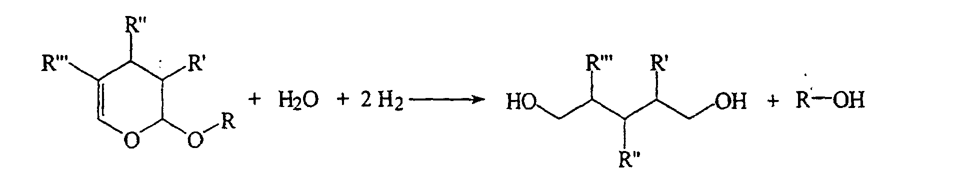

In den allgemeinen Formeln (I) bzw. (II) können R, R', Rb", R''' gleich oder verschieden sein und Wasserstoff oder einen linearen oder verzweigten gesättigten Kohlenwasserstoffrest bedeuten. Bevorzugte Kohlenwasserstoffreste sind C₁-C₈-Alkyl wie Methyl, Ethyl, n-Propyl, Iso-Propyl, n-Butyl, Iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl und 2-Ethylhexyl, besonders bevorzugt sind die genannten C₁-C₄-Alkylreste.

Die Kohlenwasserstoffreste können in der Kohlenwasserstoffkette als Heteroatome O, S und N enthalten. Beispiele sind C₂-C₂₀-Alkoxyalkyl, bevorzugt C₂-C₈-Alkoxyalkyl, besonders bevorzugt C₂-C₄-Alkoxyalkyl wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl, C₂-C₂₀-Alkylflaioalkyl, wie die den genannten Alkoxyalkylresten entsprechenden Alkylthioalkylreste, C₃-C₂₀-Dialkylaminoalkyl, bevorzugt C₃-C₁₀-Dialkylaminoalkyl wie Dimethylaminomethyl, Diethylaminoethyl, Di-n-propylaminoethyl und Diisopropylaminoethyl, C₂-C₂₀-Alkylaminoalkyl, bevorzugt C₂-C₈-Alkylaminoalkyl wie Methylaminomethyl, Methylaminoethyl, Ethylaminomethyl, Ethylaminoethyl und iso-Propylaminoethyl.

Die genannten Kohlenwasserstoffreste können mit Hydroxy-, Thio-, Aminogruppen oder Halogenen einfach oder mehrfach substituiert sein.

Besonders bevorzugt werden Alkoxydihydropyrane der allgemeinen Formel (IIa) 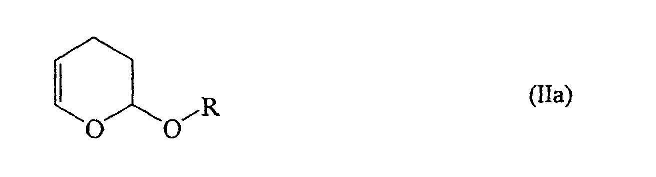 wobei R lineares oder verzweigtes C₁-C₄-Alkyl bedeutet, eingesetzt.

Insbesondere werden folgende Alkoxydihydropyrane eingesetzt:

2-Methoxy-2,3-dihydro-4H-pyran, 2-Ethoxy-2,3-dihydro-4H-pyran, 2-Propoxy-2,3-dihydro-4H-pyran, 2-Butoxy-2,3-dihydro-4H-pyran und 2-Isobutoxy-2,3-dihydro-4H-pyran.

Das erfindungsgemäße Verfahren wird vorzugsweise bei einer Temperatur von 50 bis 250°C, besonders bevorzugt von 100 bis 200°C und einem Druck von vorzugsweise 10 bis 300 bar absolut, besonders bevorzugt von 50 bis 220 bar absolut, durchgeführt. Der Druck wird durch Zugabe von Wasserstoff aufrecht erhalten. Das Verfahren kann kontinuierlich oder diskontinuierlich durchgeführt werden, wobei in beiden Fällen Wasserstoff im Kreis geführt werden kann. Der Reaktor kann in Suspensions- oder Festbettfahrweise betrieben werden. Dabei werden Wasser und das Alkoxydihydropyran flüssig dem Reaktor zugeführt, bevorzugt in einem Molverhältnis von Wasser zu Alkoxydihydropyran von 1,5 : 1 bis 10 : 1, besonders bevorzugt von 3 : 1 bis 8 : 1, bezogen auf den frisch zugeführten Reaktoreintrag. Dabei wird der Katalysator im allgemeinen mit 0,1 bis 5,0 kg, bevorzugt mit 0,3 bis 1,5 kg Alkoxydihydropyran pro Kilogramm Katalysator und Stunde belastet.

Bevorzugt wird das erfindungsgemäße Verfahren kontinuierlich durchgeführt, wobei der Reaktor vorzugsweise in Festbettfahrweise betrieben wird. Der Reaktor kann sowohl in der Sumpf- als auch in der Rieselfahrweise betrieben werden, d.h. das Katalysatorbett kann sowohl von oben nach unten als auch von unten nach oben von dem flüssigen Reaktoreintrag durchströmt werden. Dabei kann Wasserstoff sowohl im Gleichstrom mit oder im Gegenstrom zum flüssigen Reaktoreintrag geführt werden.

Bei kontinuierlicher Durchführung des Verfahrens wird bei stationärer Betriebsweise des Reaktors vorzugsweise ein Teil des flüssigen Reaktoraustrages dem Reaktor erneut zugeführt, wobei besonders bevorzugt pro Kilogramm des frisch zugeführten Alkoxydihydropyrans 2 bis 10 kg des flüssigen Reaktoraustrages dem Reaktor erneut zugeführt werden.

Hinter dem Reaktor ist vorzugsweise ein Abscheider angebracht, in welchem der Reaktoraustrag entspannt und Wasserstoffgas und Flüssigkeit voneinander getrennt werden. Im allgemeinen wird der überwiegende Teil des Wasserstoffs, vorzugsweise 70 bis 95 Volumen-%, in den Reaktor zurückgeführt. Der entnommene flüssige Reaktoraustrag kann in an sich bekannter Weise aufgearbeitet werden. Bevorzugt wird er einer zweistufigen Rektifikation unterworfen, wobei in der ersten Stufe Wasser und der entstandene Alkohol, beispielsweise Methanol, abgetrennt und in der zweiten Stufe bei vermindertem Druck reines 1,5-Pentandiol gewonnen wird.

Die Erfindung wird durch die nachstehenden Beispiele näher erläutert.

### Beispiele

### Herstellung der Katalysatoren

Die Herstellung der in dem erfindungsgemäßen Verfahren eingesetzten Katalysatoren A und B erfolgt gemäß dem in EP-A 0 394 842 beschriebenen Verfahren.

### Katalysator A:

Nickelnitrat, Kupfernitrat und Zirkonacetat werden in destilliertem Wasser gelöst, wobei eine Lösung erhalten wird, welche umgerechnet 4,48 Gew.-% NiO, 1,52 Gew.-% CuO und 2,8 Gew.-% ZrO₂ enthält. Der Lösung wird zur Fällung in einem Rührgefäß ein konstanter Strom einer 20%igen wäßrigen Carbonatlösung bei einer Temperatur von 70°C zugesetzt, so daß bei der Fällung ein pH-Wert des Gemisches von 7,0 aufrecht erhalten wird. Die erhaltene Suspension wird filtiert und der Filterkuchen so lange mit destilliertem Wasser gewaschen, bis das Filtrat eine elektrische Leitfähigkeit von 20 µS aufweist. Anschließend wird Ammoniumheptamolybdat entsprechend einer Menge von 30 g MoO₃ pro kg NiO in den wasserfeuchten Filterkuchen eingearbeitet und der Filterkuchen bei einer Temperatur von 150°C getrocknet. Das so erhaltene Hydroxid/Carbonat-Gemisch wird nachfolgend bei einer Temperatur von 500°C über einen Zeitraum von 4 Stunden getempert. Die erhaltene Katalysatormasse besteht aus 50 Gew.-% NiO, 17 Gew.-% CuO, 1,5 Gew.-% MoO₃ und 31,5 Gew.-% ZrO₂. Das Katalysatorpulver wird mit 3 Gew.-% Graphit vermischt und zu Tabletten von 6 mm Durchmesser und 3 mm Dicke verpreßt. Die Tabletten haben eine Porosität von 0,30 ml/g und eine Härte von 3500 N/cm².

### Katalysator B:

Es wird wie bei der Herstellung von Katalysator A verfahren, jedoch kein Ammoniumheptamolybdat zugegeben. Der so erhaltene Katalysator besteht aus 51 Gew.-% NiO, 17 Gew.-% CuO und 32 Gew.-% ZrO₂. Das Katalysatorpulver wird wie oben angegeben mit Graphit vermischt und zu Tabletten verpreßt.

### Herstellung von 1,5-Pentandiol

### Vergleichsbeispiel V1 (gem. US 2,546,019)

In einem Rührautoklaven mit einem Gesamtvolumen von 300 ml wurden 144,82 g (= 1,269 mol) 2-Methoxy-2,3-dihydro-4H-pyran (MOP), 45,76 g (= 2,54 mol) Wasser und 9,4 g Raney-Nickel gegeben und 100 bar Wasserstoff aufgepreßt. Der Reaktor wurde unter Rühren auf ein 150°C aufgeheizt und 4 Stunden bei dieser Temperatur gehalten. Anschließend wurde der Reaktor abgekühlt und auf Normaldruck entspannt. Der Reaktorinhalt wurde filtriert, wobei 190 g Filtrat erhalten wurden. In dem Filtrat wurden mittels GC-Analyse die organischen Bestandteile und mittels Karl-Fischer-Titration der Wassergehalt bestimmt. Es wurde eine Pentandiol-Konzentration von 48,1 Gew.-% ermittelt, was einer Gesamtmenge von 91,7 g Pentandiol (= 0,88 mol) entspricht. Die Ausbeute betrug somit, bezogen auf eingesetztes MOP, 69 mol-%. Die Raum-Zeit-Ausbeute betrug 120 g/(l x h).

### Beispiele 1 und 2

Es wurde wie im Vergleichsbeispiel verfahren, jedoch unter Verwendung der Katalysatoren A und B an Stelle von Raney-Nickel. Es wurden jeweils 9,4 g feingemahlener Katalysator eingesetzt.

Die Ergebnisse sind in der nachstehenden Tabelle zusammengefaßt.

| Beispiel Nr. | V1 | 1 | 2 |
|---|---|---|---|
| Katalysator | Raney-Ni | Kat. A | Kat. B |
| Katalysator [g] | 9,4 | 9,4 | 9,4 |
| Umsatz bzgl. MOP [%] | 99,95 | 99,97 | 100,00 |
| PDO im Austrag [Gew.-%] | 48,15 | 57,3 | 53,2 |
| Ausbeute Pentandiol [g] | 91,7 | 109,3 | 101,3 |
| Raum-Zeit-Ausbeute [g/ (l*h)] | 120,2 | 143,3 | 132,9 |
| Selektivität bzgl. MOP [mol/mol] | 69,4% | 82,7% | 76,7% |

Wie die Tabelle zeigt, wird mit den erfindungsgemäßen Katalysatoren eine deutlich höhere Selektivität der Umsetzung zu 1,5-Pentandiol und eine deutlich höhere Raum/Zeit-Ausbeute als mit Raney-Nickel erzielt.

### Kontininuierliche Umsetzung

### Beispiel 3:

Für eine kontinuierliche Verfahrensführung wird ein Hochdruckreaktor mit 30 mm Innendurchmesser und 2000 mm Gesamthöhe eingesetzt. Im Reaktor ist ein Thermoelement mit 12 mm Durchmesser in axialer Richtung angeordnet. Der Reaktor wird im unteren Teil mit 500 ml Pallringen aus Edelstahl gefüllt, darüber werden 500 ml (= 760 g) des Katalysators A in Tablettenform eingefüllt und als oberste Schüttung 250 ml Pallringe eingefüllt. Dem Reaktor werden am Boden über jeweils getrennte Leitungen 250 g/h 2-Methoxy-2,3-dihydro-4H-pyran (MOP), 250 g/h Wasser sowie 500 g/h des flüssigen Reaktionsaustrages zugeführt. Am Boden des Reaktors wurde Wasserstoff zugegeben, wobei die Wasserstoffmenge so geregelt wurde, daß die Abgasmenge konstant bei 100 Nl/h lag. Der Reaktor wurde bei 150°C und 200 bar absolut Wasserstoff und Sumpffahrweise betrieben. Der Reaktoraustrag wurde abgekühlt und auf Normaldruck entspannt, der nicht zurückgeführte Teil des Reaktoraustrages wurde als Rohprodukt abgezogen.

Das Rohprodukt enthielt 42 Gew.-% Wasser, 1,6 Gew.-% Tetrahydropyran, 8,7 Gew.-% Methanol, 47,5 Gew.-% 1,5-Pentandiol sowie 0,2 Gew.-% weitere organische Nebenprodukte. Der Umsatz bezüglich MOP betrug 100%, die Ausbeute an 1,5-Pentandiol betrug bezogen auf eingesetztes MOP 96,4%.

Das Rohprodukt (500 g) wurde mittels einfacher Destillation zunächst bei Normaldruck entwässert, wobei 259 g Destillat (Wasser und Methanol) anfielen und anschließend bei 30 mbar im Vakuum destilliert, wobei 220,5 g Pentandiol mit einer Reinheit von 99 Gew.-% erhalten wurden. Die Destillationsausbeute betrug somit 92,8 Gew.-% des im Rohprodukt enthaltenen 1,5-Pentandiols, die Gesamtausbeute bezogen auf eingesetztes MOP lag bei 89,5%. Die Raum-Zeit-Ausbeute für das im Rohprodukt enthaltene 1,5-Pentandiol bezogen auf das Katalysatorvolumen lag bei 475 g/(l x h).

## Patentansprüche

1. Verfahren zur Herstellung von 1,5-Pentandiolen der allgemeinen Formel (I) 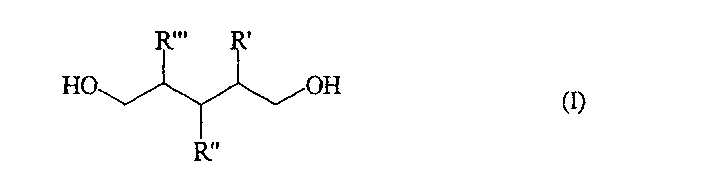 durch einstufige Umsetzung von Alkoxydihydropyranen der allgemeinen Formel (II) 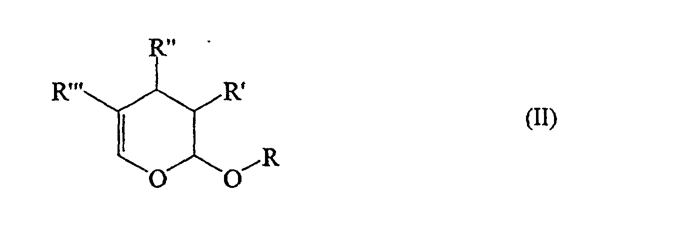 wobei in den allgemeinen Formeln (I) bzw. (II)
R, R', R'', R''' gleich oder verschieden sein können und Wasserstoff oder einen linearen oder verzweigten gesättigten Kohlenwasserstoffrest mit 1 bis 20 C-Atomen, der in der Kohlenwasserstoffkette als Heteroatome O, S und N enthalten kann und mit Hydroxy-, Thiol-, Aminogruppen oder Halogenen einfach oder mehrfach substituiert sein kann,
bedeuten,
mit Wasser und Wasserstoff in Gegenwart eines Katalysators, **dadurch gekennzeichnet, daß** der Katalysator Oxide von Nickel, Zirkon und Kupfer enthält.

2. Verfahren nach Anspruch 1, **dadurch gekennzeichnet, daß** der Katalysator
- 20 bis 75 Gew.-% NiO,
- 10 bis 75 Gew.-% ZrO₂,
- 5 bis 50 Gew.-% CuO und
- 0 bis 5 Gew.-% MoO₃,
wobei die Summe der genannten Oxide 100 Gew.-% ergibt, enthält.

3. Verfahren nach Anspruch 1 oder 2, **dadurch gekennzeichnet, daß** Alkoxydihydropyrane der allgemeinen Formel (IIa) 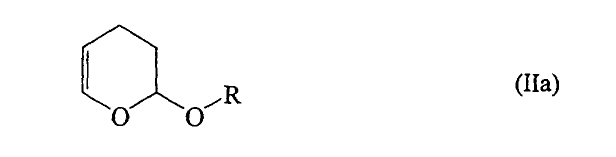 wobei
R lineares oder verzweigtes C₁-C₄-Alkyl bedeutet, eingesetzt werden.

4. Verfahren nach einem der Ansprüche 1 bis 3, **dadurch gekennzeichnet, daß** die Umsetzung bei einer Temperatur von 50 bis 250 °C und einem Druck von 10 bis 300 bar absolut durchgeführt wird.

5. Verfahren nach einem der Ansprüche 1 bis 4, **dadurch gekennzeichnet, daß** einem Reaktor Wasser und Alkoxydihydropyran in einem Molverhältnis von Wasser zu Alkoxydihydropyran von 1,5 : 1 bis 10 : 1 frisch zugeführt wird.

6. Verfahren nach einem der Ansprüche 1 bis 5, **dadurch gekennzeichnet, daß** die Umsetzung in einem Reaktor in Suspensions- oder Festbettfahrweise kontinuierlich mit einer Katalysatorbelastung von 0,1 bis 5,0 kg Alkoxydihydropyran (kg_{Katalysator} x h) durchgeführt wird.

7. Verfahren nach Anspruch 6, **dadurch gekennzeichnet, daß** bei stationärer Betriebsweise des Reaktors ein Teil des flüssigen Reaktoraustrages dem Reaktor erneut zugeführt wird, wobei pro Kilogramm des frisch zugeführten Alkoxydihydropyrans 2 bis 10 kg des flüssigen Reaktoraustrages erneut zugeführt werden.

8. Verfahren nach Anspruch 6 oder 7, **dadurch gekennzeichnet, daß** die Umsetzung in Festbettfahrweise durchgeführt wird, wobei das Katalysatorbett den Katalysator als lose Schüttung von Katalysatorformkörpern enthält und von oben oder von unten von dem flüssigen Reaktoreintrag durchströmt wird, wobei Wasserstoff im Gleichoder Gegenstrom geführt werden kann.

9. Verwendung eines Katalysators, wie er in Anspruch 1 oder 2 definiert ist, in einem Verfahren zur Herstellung von 1,5-Pentandiolen, wie es in einem der Ansprüche 1 bis 8 definiert ist.

## Claims

1. A process for preparing 1,5-pentanediols of the formula (I) 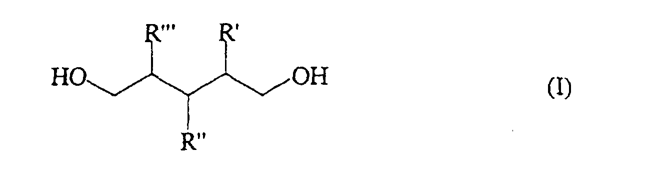 by single-stage reaction of alkoxydihydropyrans of the formula (II) 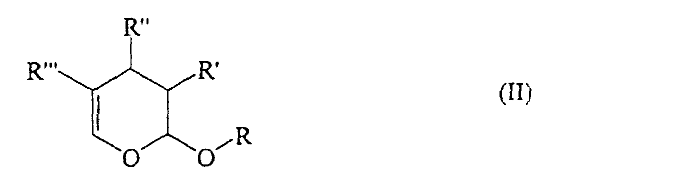 where, in the formulae (I) and (II),
R, R', R", R"' can be identical or different and are each hydrogen or a linear or branched saturated hydrocarbon radical having from 1 to 20 carbon atoms in which the hydrocarbon chain may contain O, S and N as heteroatoms and which may be monosubstituted or polysubstituted by hydroxy, thiol or amino groups or halogens,
with water and hydrogen in the presence of a catalyst comprising oxides of nickel, zirconium and copper.

2. A process as claimed in claim 1, wherein the catalyst comprises
- from 20 to 75% by weight of NiO,
- from 10 to 75% by weight of ZrO₂,
- from 5 to 50% by weight of CuO and
- from 0 to 5% by weight of MoO₃,
where the sum of the oxides specified is 100% by weight.

3. A process as claimed in claim 1 or 2, wherein alkoxydihydropyrans of the formula (IIa) 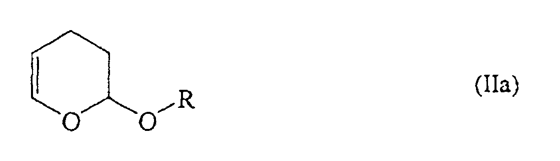 where
R is linear or branched C₁-C₄-alkyl, are used.

4. A process as claimed in any of claims 1 to 3, wherein the reaction is carried out at from 50 to 250°C and a pressure of from 10 to 300 bar absolute.

5. A process as claimed in any of claims 1 to 4, wherein water and alkoxydihydropyran are present in the fresh feed to a reactor in a molar ratio of water to alkoxydihydropyran of from 1.5:1 to 10:1.

6. A process as claimed in any of claims 1 to 5, wherein the reaction is carried out continuously in a reactor in suspension or fixed-bed mode at a throughput over the catalyst of from 0.1 to 5.0 kg of alkoxydihydropyran/(kg_{catalyst} × h).

7. A process as claimed in claim 6, wherein, in steady-state operation of the reactor, part of the liquid reactor product is recirculated to the reactor in an amount of from 2 to 10 kg for every kilogram of fresh alkoxydihydropyran fed in.

8. A process as claimed in claim 6 or 7, wherein the reaction is carried out in the fixed-bed mode and the catalyst is present as a loose bed of shaped catalyst bodies through which the liquid reactor feed flows from the top downward or from the bottom upward, with the hydrogen being able to be passed through in cocurrent or countercurrent.

9. The use of a catalyst as defined in claim 1 or 2 in a process for preparing 1,5-pentanediols as defined in any of claims 1 to 8.

## Revendications

1. Procédé de préparation de 1,5-pentanediols de la formule générale (I) : 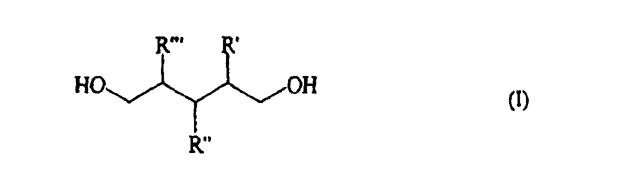 par réaction en une étape d'alcoxydihydropyrannes de la formule générale (II) : 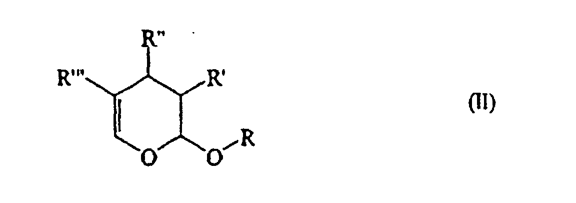 dans lesquelles formules générales (I) et respectivement (II)
R, R', R", R''' peuvent être identiques ou différents et représentent de l'hydrogène ou un radical d'hydrocarbure saturé, linéaire ou ramifié, comportant 1 à 20 atomes de C, qui peut contenir, dans la chaîne hydrocarbonée, O, S et N comme hétéroatomes et qui peut être substitué à une ou à plusieurs reprises par des groupes hydroxy, thiol ou amino ou par des halogènes,
avec de l'eau et de l'hydrogène en présence d'un catalyseur, **caractérisé en ce que** le catalyseur contient des oxydes de nickel, de zirconium et de cuivre.

2. Procédé suivant la revendication 1, **caractérisé en ce que** le catalyseur contient :
- 20 à 75% en poids de NiO,
- 10 à 75% en poids de ZrO₂,
- 5 à 50% en poids de CuO, et
- 0 à 5% en poids de MoO₃,
la somme des oxydes cités donnant 100% en poids.

3. Procédé suivant l'une des revendications 1 et 2, **caractérisé en ce qu'**on met en oeuvre des alcoxydihydropyrannes de la formule générale (IIa) : 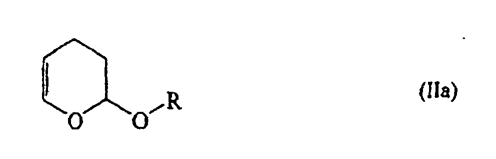 dans laquelle
R représente un groupe alkyle en C₁-C₄ linéaire ou ramifié.

4. Procédé suivant l'une des revendications 1 à 3, **caractérisé en ce que** la réaction est effectuée à une température de 50 à 250°C et à une pression absolue de 10 à 300 bars.

5. Procédé suivant l'une des revendications 1 à 4, **caractérisé en ce qu'**à un réacteur on amène, à l'état frais, de l'eau et de l'alcoxydihydropyranne dans un rapport molaire entre eau et alcoxydihydropyranne de 1,5/1 à 10/1.

6. Procédé suivant l'une des revendications 1 à 5, **caractérisé en ce que** la réaction est effectuée dans un réacteur en continu, selon un mode de fonctionnement en suspension ou à lit fixe, avec une charge au catalyseur de 0,1 à 5,0 kg d'alcoxydihydropyranne (kg_{catalyseur} x h).

7. Procédé suivant la revendication 6, **caractérisé en ce que**, dans un mode de fonctionnement stationnaire du réacteur, une partie de l'effluent liquide du réacteur est ramenée au réacteur, 2 à 10 kg de l'effluent liquide du réacteur étant ramenés par kilogramme de l'alcoxydihydropyranne alimenté à l'état frais.

8. Procédé suivant l'une des revendications 6 et 7, **caractérisé en ce que** la réaction est effectuée dans un mode de fonctionnement à lit fixe, le lit de catalyseur contenant le catalyseur sous la forme d'une masse incohérente de corps façonnés de catalyseur et étant traversé à partir du haut ou à partir du bas par le produit d'entrée liquide du réacteur, de l'hydrogène pouvant être conduit dans un écoulement dans le même sens ou en contre-courant.

9. Utilisation d'un catalyseur tel que défini dans la revendication 1 ou 2, dans un procédé de préparation de 1,5-pentanediols, comme défini dans une des revendications 1 à 8.
